# EUROPÄISCHE PATENTSCHRIFT

(11) **EP 3 167 891 B1**
(45) Veröffentlichungstag und Bekanntmachung des Hinweises auf die Patenterteilung: **23.01.2019**
(21) Anmeldenummer: 16455004.8
(22) Anmeldetag: 25.10.2016
(51) Int. Cl.: A61K 33/08, A61K 45/06, A61K 9/00, A61K 9/14, A61P 15/02, A61P 31/04, A61P 31/10

(54) **BEHANDLUNG VON VAGINITIS**
TREATMENT OF VAGINITIS
TRAITEMENT DE LA VAGINITE

(30) Priorität: 12.11.2015 EP 15194288
(43) Veröffentlichungstag der Anmeldung: 17.05.2017
(73) Patentinhaber: Glock Health, Science and Research GmbH, 2232 Deutsch Wagram (AT)
(72) Erfinder: Glock, Gaston, 9220 Velden (AT)
(74) Vertreter: Patentanwälte Barger, Piso & Partner

(56) Entgegenhaltungen:
- EP-A1- 0 956 858
- EP-A1- 2 040 837
- WO-A1-00/64586
- WO-A1-2010/057849
- WO-A2-2007/054085

## Beschreibung

Die Erfindung betrifft die Behandlung von Vaginalmykosen, bakteriellen Vaginosen und anderer Formen der Vaginitis (Scheidenentzündung) entsprechend dem Anspruch 1.

Aus der WO 00/64586 ist eine Vorrichtung und ein damit zusammenhängendes Verfahren zum Zerkleinern mineralischen Materials bekannt, von dem in der Folge kursorisch aufgezählt wird, bei welchen Krankheiten es erfolgreich einsetzbar ist, wobei fast keine Krankheit ausgelassen wird. Es wurden einige Verweise auf Tests betreffend karzinogene Erkrankungen, Lebererkrankungen Diabetes, Multipler Sklerose gegeben, ohne jeden Hinweis auf die durchführende Organisation oder irgendwelche Details. Weitere genannte Anwendungsgebiete sind die Herstellung von Lebensmitteln, von Kosmetika, von Pflanzenschutzmitteln, von Zigaretten, und Rohstoffen in der Bauindustrie. Die beschriebene Vorrichtung schafft es, über 98 % aller Teilchen auf unter 4,3 µm zu zerkleinern, wobei über 28 % aller Teilchen kleiner als 0,5 µm sind. Diese Angaben stehen zu denen der Anwendung in keiner Beziehung.

Vom gleichen Erfinder stammt die WO 2007/054085, die offenbart, auf unter 100 Nanometer, entspricht 0,1 µm, zerkleinerten Klinoptilolith mit Propolis und/oder Kolostrum als antivirales Mittel, unter anderem zur Behandlung von Influenza, Schnupfen, Husten, Masern, Mumps, Röteln, Ringelröteln, Drei-Tage-Fieber, Windpocken, Pfeiffer'sches Drüsenfieber, SARS, Zytomegalie, Durchfall, Hepatitis, Kinderlähmung, Herpes labialis, Warzen, Tollwut, Lassa-Fieber, Ebola, Marburg-Fieber, Hanta-Virus-Fieber, FSME, RSSE, Louping-ill-Enzephalitis, Powassan-Enzephalitis, Kyasanur-forest-Fieber, Omsk-hämorrhagisches Fieber, Colorado-tick-Fieber, Gelbfieber, Dengue-Fieber, Japanische Enzephalitis, West-Nil-Fieber, Chikungunya-Fieber, Q'nyong-nyong-Fieber, Rift-Tal Fieber, SandmOcken-Fieber, Ross-River-Fieber, Sindbis-Fieber, Mayaro-Fieber, Murray-Valley-Enzephalitis, St. Louis-Enzephalitis, Rocio-Enzephalitis, California-Enzephalitis, Bunyamwera-Fieber, Oropouche-Fieber, AIDS, Herpes genitalis und/oder Herpes simplex einzusetzen. Im Zuge der "Behandlung" von AIDS wird als auftretendes klinisches Bild unter vielem andren auch vaginale Candidose genannt. Klinische Tests werden nicht erwähnt, das alles ist reines Wunschdenken.

Die WO 2008/003101 des Erfinders der vorliegenden Anmeldung offenbart ein Verfahren zur Reinigung von Klinoptilolith von Schwermetallen.

Die WO 2010/057849 beschreibt die Verbesserung der Wundheilung durch Aufbringen zerkleinerten Klinoptiloliths, der die Konzentration von Aminen reduziert. Ohne näheren Bezug zu den einzelnen Anwendungen werden Partikelgrößen von 2 bis 16 µm genannt, die auch über ein vorgegebenes Zeta-Potential und eine vorgegebene spezifische Oberfläche verfügen müssen, Messmethoden werden nicht angegeben. Als Beispiele werden ausschließlich in-vitro-Versuche mit künstlich erstellten aminhältigen wässerigen Lösungen angegeben.

Aus der EP 956 858 ist es bekannt, dass probiotische Bakterien bei der Behandlung von vaginalen Infektionen nützlich sind.

Allgemein ist folgendes auszuführen:
Im typischen (gesunden) Fall setzt sich das vaginale Mikrobiom der geschlechtsreifen Frau größtenteils aus unterschiedlichen Laktobazillen (je nach ethnischer Zugehörigkeit *Lactobacillus crispatus, L. gasseri, L. iners, L. jensenii*) zusammen, die u.a. für einen optimalen pH-Wert sorgen und das Wachstum von Pathogenen unterdrückt. Unter dem Einfluss verschiedener Umweltfaktoren wird die natürliche Vaginalflora angegriffen und es kann infolge eines Ungleichgewichts (Dysbiose) zu einem Überhandnehmen pathogener Keime kommen.

Es ist Ziel und Aufgabe der Erfindung, eine Behandlung anzugeben, die einerseits die pathogenen Keime möglichst effektiv bekämpft, andererseits die nützlichen Laktobazillen möglichst schont.

Diese erfindungsgemäße Aufgabe wird durch die im Anspruch 1 angegebenen Merkmale gelöst; mit anderen Worten, es wird Klinoptilolith, der eine Partikelgröße zwischen 0,2 und 10 µm aufweist lokal (äußerlich) zur Behandlung von Vaginalerkrankungen wie Bakterieller Vaginose, Vulvovaginaler Candidose und Trichomoniasis von Säugetieren und Menschen sowie zur Wiederherstellung des gesunden Vaginalmikrobioms eingesetzt. Für Jurisdiktionen, in denen dies möglich oder gefordert ist, besteht die Erfindung darin, dass Klinoptilolith, der eine Partikelgröße zwischen 0,2 und 10 µm aufweist, zur Herstellung eines Medikamentes zur Behandlung der eingangs genannten Erkrankung(en) verwendet wird.

Bevorzugt ist der Klinoptilolith von Schwermetallen befreit, besonders bevorzugt durch ein Verfahren entsprechend dem in der EP 2 040 837 bzw. der korrespondierenden US 8,173,101 angegebenen Verfahren. Für Jurisdiktionen, in denen dies möglich ist, wird der Offenbarungsgehalt dieser Druckschriften durch Bezugnahme zum Inhalt der vorliegenden Anmeldung gemacht.

Die Wirksamkeit der erfindungsgemäßen Substanz geht aus den nachfolgend geschilderten Untersuchungen und Experimenten an mehreren unterschiedlichen Mikroorganismen-Stämmen hervor. Es wird belegt, dass die Substanz auf Vertreter der Gattung Candida sowie auf ausgewählte bakterielle Pathogene der Vaginalflora eine wachstumshemmende Wirkung aufweist, während Vertreter einer gesunden Vaginalflora im Wachstum sogar gefördert werden.

Um den Zusammenhang zwischen der Partikelgröße und der Wirksamkeit zu veranschaulichen, sind am Ende der die Wirksamkeit belegenden Versuche entsprechende Vergleichsversuche angehängt.

Die in [n] gesetzten Hinweise beziehen sich auf die am Ende der Beschreibung angeführte Literatur. Die Ergebnisse sind in verschiedenen Tabellen und Graphiken in Form von Figuren dargestellt, dabei zeigt bzw. zeigen:
- die Fig. 1: das Wachstum von *Candida albicans* mit/ohne Zeolith in Sab-Bouillon,
- die Fig. 2: den Einfluss von Zeolith auf das Wachstum von *Candida albicans* in Sabouraud Bouillon,
- die Fig. 3: den Einfluss von Zeolith auf die Keimzahl von *Candida albicans* Kulturen nach 300 min. Inkubation,
- die Fig. 4: den Einfluss von Zeolith auf das Wachstum von *Candida albicans* in Sabouraud Bouillon (Vialight Assay),
- die Fig. 5: den Einfluss von Zeolith auf das Wachstum von *Candida parapsilosis* in Sabouraud Bouillon,
- die Fig. 6: die Wirkung von Zeolith auf das Wachstum von *Candida parapsilosis* in Sabouraud Bouillon,
- die Fig. 7: den Einfluss von Zeolith auf das Wachstum von *Gardnerella vaginalis* Viabilitätsmessung mittels Vialight Assay nach 15 Stunden Inkubation,
- die Fig. 8: den Einfluss von Zeolith auf das Wachstum von *G*. *vaginalis* Viabilitätsmessung mittels Vialight Assay nach 15 Stunden Inkubation,
- die Fig. 9: den Einfluss von Zeolith auf das Wachstum von *L. crispatus* - ATP-Messung nach 6 bzw. 24 Stunden Inkubation,
- die Fig. 10: die Zellzahl der *L. crispatus* Kulturen nach 24 Stunden Inkubation, durch Koloniezählung ermittelt,
- die Fig. 11: die pH-Werte der Medien und Kulturen von *L. crispatus* nach 24 Stunden Inkubation,
- die Fig. 12: den Einfluss von Zeolith auf das Wachstum von *L. jensenii* in MRS Bouillon binnen 3er Stunden,
- die Fig. 13: den Einfluss von Zeolith auf das Wachstum von *L. jensenii* in MRS Bouillon binnen 4 und 7 Stunden,
- die Fig. 14: den Einfluss von Zeolith auf das Wachstum von *Lactobacillus casei* in MRS-Bouillon - Resazurinassay,
- die Fig. 15: den Einfluss von Zeolith auf das Wachstum von *Lactobacillus casei* in MRS-Bouillon - Vialight Assay,
- die Fig. 16: die Lactat-Gehalte versch. Kulturen und Co-Kulturen nach 6 Stunden Inkubation,
- die Fig. 17: die Lactat-Gehalte versch. 24-Stunden alter Kulturen und Co-Kulturen,
- die Figs. 18-20: Agarplatten mit unterschiedlichen Kulturen,
- die Figs. 21-23: Partikelgrößenverteilungen die bei den Vergleichsversuchen verwendet wurden,
- die Figs. 24 und 25: das von der Partikelgrößenverteilung abhängige Wachstum von *C. albicans* - Resazurinmessung,
- die Figs. 26-28: das Gleiche für *C*. *glabrata* und
- die Fig. 29: das von der Partikelgrößenverteilung abhängige Wachstum von C. glabrata - Vialight Assay.

### Zu den Erkrankungen und ihrer Behandlung:

### Vaginalmykose:

Die Vaginalmykose stellt nach der bakteriellen Vaginose die zweithäufigste Ursache einer Infektion der Scheide dar.

Hauptverursacher für bestätigte Infektionen sind Hefepilze der Gattung Candida, wobei *Candida albicans* für 85-90 % aller Scheidenpilzinfektionen verantwortlich ist. Risikofaktoren, die eine Vaginalmykose begünstigen sind:
Schwangerschaft
Antibiotikaeinnahme
Diabetes mellitus
Immunsuppressiva
Medikamente einer Krebstherapie
Kortisonhältige Medikamente
Stress oder psychische Belastung

Empfohlene Therapien im Stand der Technik bestehen in der topischen Anwendung unterschiedlicher Antimykotika wie Polyenen (Nystatin), Imidazolen (Butoconazol, Clotrimazol etc.) oder Ciclopiroxolamin. Diese werden in einer großen Zahl verschiedener Zubereitungen wie Vaginaltabletten, Ovula oder Vaginalcremes intravaginal verabreicht. Die Behandlungsdauer kann 1 bis 7 Tage betragen. Eine weitere Möglichkeit besteht in der einmaligen Anwendung eines Triazolwirkstoffes (Fluconazol, Itraconazol). Parallel zur Antimykotikatherapie kann die lokale Gabe von Probiotika (z.B. Gynoflor, Multi-Gyn Flora plus u.v.m.) unterstützen, die gesunde Vaginalflora wieder aufzubauen und den pH-Wert des Milieus zu senken. Diese Produkte (Vaginaltabletten, Ovula, Gele, Cremes etc.) können unter anderem entweder lebensfähige Lactobacilli (sog. "Döderlein Stäbchen") oder Lysate davon enthalten, sowie Estriol, das als Östrogenderivat den Aufbau eines "gesunden" Schleimhautmilieus fördert, und Lactose (Milchzucker) als Nährstoff für die Lactobacilli.

In Fällen einer lokalen immunologischen Schwäche tritt bereits kurz nach Beendigung der Therapie ein Rückfall ein. Gegen diese chronisch rezidivierende *Candida albicans* Vulvovaginitis werden in Ermangelung an Alternativen lokale oder orale Erhaltungstherapien empfohlen, um Rückfälle zu vermeiden. Hier wird als Initialtherapie 7-14 Tage lang ein topisches Produkt wie zuvor beschrieben angewandt und parallel dazu oral in der ersten Woche 3 x 200mg Fluconazol verabreicht. Die Erhaltungstherapie besteht im Anschluss daran aus einer wöchentlichen Gabe von 150mg Fluconazol.

Sämtliche Konzentrationsangaben in der Beschreibung, den Ansprüchen und den Figura sind, wenn nicht anders angeführt, als mg/ml bzw als % M/V [kg/Liter] zu sehen.

Erfindungsgemäß wird Klinoptilolith, der eine Partikelgröße zwischen 0,2 und 10 µm aufweist, lokal in einer Konzentration von 1 mg/ml bis 10 mg/ml {entsprechend 0,1% M/V bis 1% M/V, wenn M in kg und V in Liter angegeben wird} angewandt, mit folgender Wirkung auf *Candida albicans*:

### 1. Versuch:

*Candida albicans* (DSM Nr. 1386) [4] wurde gemäß in der Routine häufig verwendeter Methoden auf Yeast Glucose Chloramphenicol (YGC) Agar herangezogen und danach in Flüssigmedium (Sabouraud Bouillon) überführt.
Nach Bestimmung der Zellzahl mittels Thoma Zählkammer wurden 12 Versuchsansätze (Kulturen) mit je 1 x 10⁵ Zellen/ml hergestellt, wobei 4 Kulturen kein Zeolithpulver zugesetzt wurde (Kontrolle), 4 Kulturen Zeolithpulver in der Menge von 1mg/ml und weiteren 4 Kulturen Zeolithpulver in der Menge von 10mg/ml zugesetzt wurde. Die Versuchskulturen wurden bei 37°C unter aeroben Bedingungen unter Schütteln inkubiert. Zur Ermittlung der Blindwerte wurden alle Medien auch ohne Keim im Versuch mitgeführt.
Über den Zeitraum von 6 Stunden wurden stündlich Aliquots der Kulturen zur Bestimmung der Stoffwechselaktivität mittels Viabilitätstest (Resazurin-Assay) [7] entnommen.

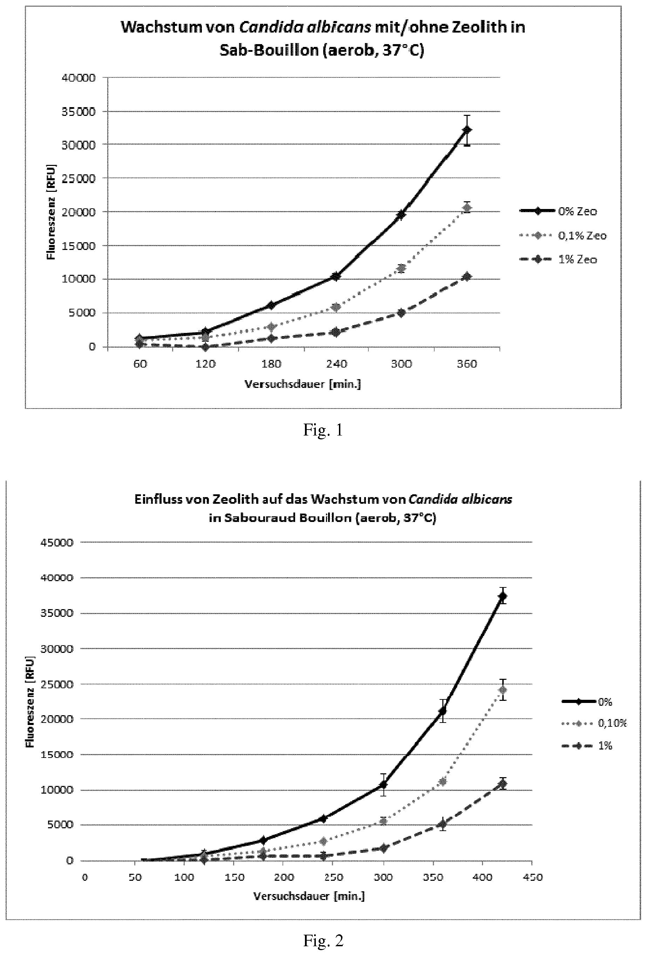

Die Ergebnisse, aus denen die hohe Wirksamkeit unmittelbar hervorgeht, sind in Fig. 1 grafisch dargestellt, Kurven von oben nach unten: 0%; 0,1% und 1% Zeolith.

### 2. Versuch:

Das zuvor beschriebene Experiment wurde mit folgenden zwei Änderungen wiederholt:
- Die Versuchsdauer wurde auf 7 Stunden ausgedehnt
- Aliquots der Kulturen wurden nach 5 Stunden Inkubation zur Bestimmung der tatsächlichen Zellzahl verdünnt, auf Sabouraud Agar ausplattiert und nach Inkubation mittels Koloniezählung ausgewertet.

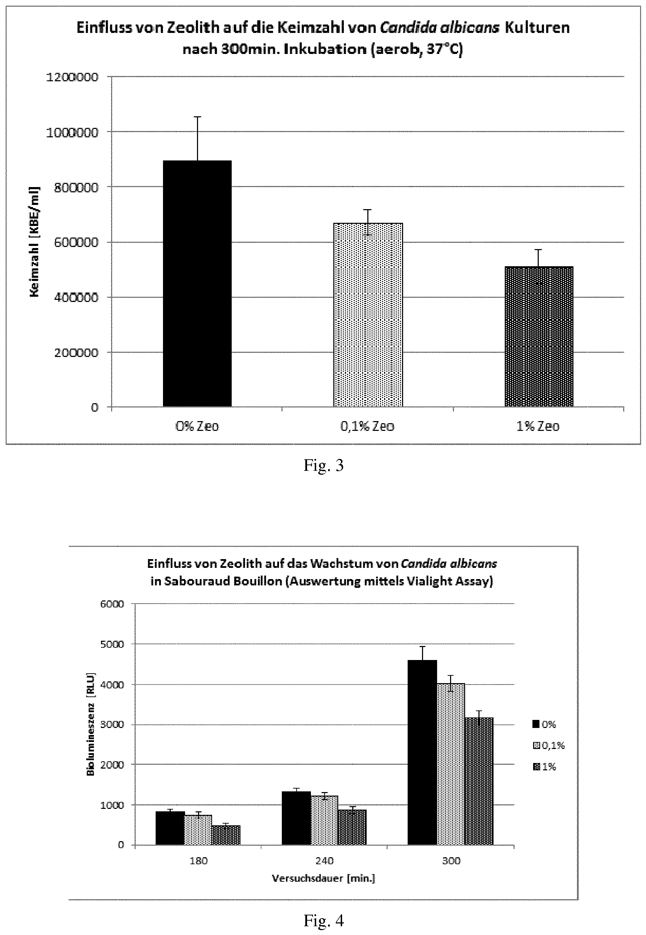

Fig. 2 zeigt die Ergebnisse des Resazurinassays, Kurven wie in Fig. 1. Das Ergebnis des vorhergehenden Wachstumsexperiments konnte bestätigt werden. In Fig. 3 sind die empirisch ermittelten Keimzahlen der Versuchsgruppen (von links: 0%, 0,1% und 1% Zeolith) grafisch dargestellt.

### 3. Versuch:

In einer weiteren Wiederholdung des Experiments wurde eine alternative Messung der Stoffwechselaktivität (Vialight Assay) gewählt [8], um die Ergebnisse der Versuche zu untermauern. Die Fig. 4 (Säulen, jeweils von links: 0, 0,1% und 1% Zeolith) zeigt die Versuchsgruppen nach 3, 4 und 5 Stunden Inkubation. Wie ersichtlich, konnte erneut mit der erfindungsgemäßen Substanz eine Verminderung der Stoffwechselaktivität von *Candida albicans* in Bouillonkultur erreicht werden.

In der Folge wurde die Wirkung der erfindungsgemäßen Substanz in Konzentrationen von 10mg/ml und 50mg/ml auf *Candida parapsilosis* (Eigenisolat; S 0811-01) [9] getestet:
*Candida parapsilosis* wurde gemäß in der Routine häufig verwendeter Methoden zunächst auf YGC-Agar herangezogen, und im Anschluss daran in Flüssigmedium (Sabouraud Bouillon) überführt und inkubiert.
Nach Bestimmung der Zellzahl mittels Zählkammer wurden 9 Versuchsansätze (Hefekulturen) mit je 1 x 10⁶ Hefe-Zellen/ml hergestellt, wobei 3 Kulturen kein Zeolithpulver zugesetzt wurde (Kontrolle), 3 Kulturen Zeolithpulver in der Menge von 10mg/ml (1% M/V) und weiteren 3 Kulturen Zeolithpulver in der Menge von 50mg/ml (5% M/V) zugesetzt wurde. Die Versuchskulturen wurden bei 30°C unter Schütteln inkubiert:
3 x 12ml Sabouraud Bouillon
3 x 12ml Sabouraud Bouillon 1% Zeolith
3 x 12ml Sabouraud Bouillon und 5% Zeolith

Zur Ermittlung der Blindwerte wurden Aliquots aller Medien auch ohne Keim inkubiert und im Versuch mitgeführt.

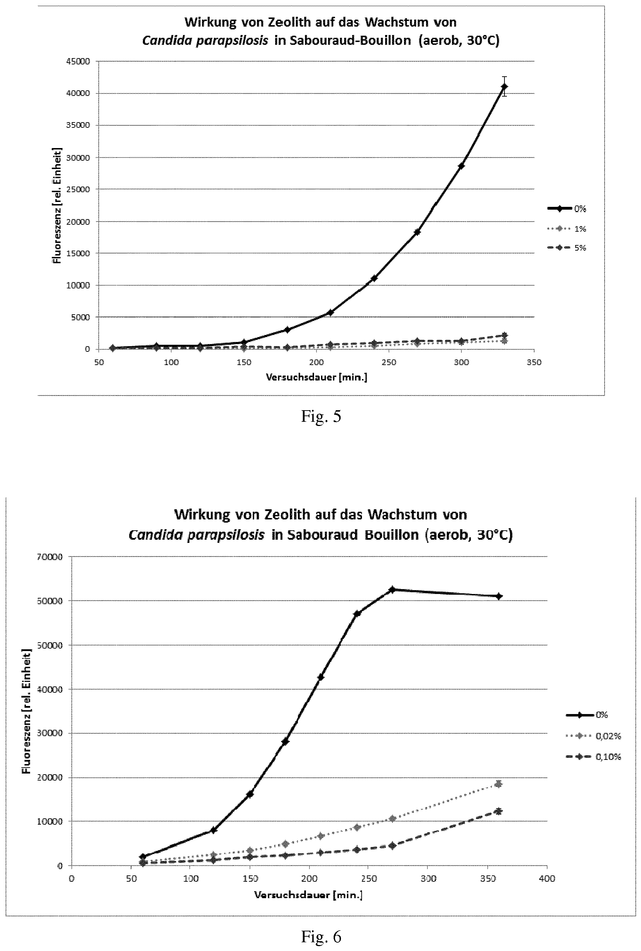

Nach 60min., 120min. und danach alle weiteren 30min. wurden die Stoffwechselaktivitäten jeder Versuchskultur indirekt mittels gängigem Viabilitätstest (Resazurinassay) bestimmt. Die Messwerte der 3 Ansätze derselben Kondition wurden gemittelt und inklusive Standardabweichung in Fig. 5 (Kurven, von oben: 0mg/ml, 10mg/ml und 50mg/ml) dargestellt.

Wie ersichtlich ist, führten beide eingesetzte Dosen der erfindungsgemäßen Substanz zu einer deutlichen Wachstumshemmung des Versuchskeims.

### 4. Versuch:

Wirkung der erfindungsgemäßen Substanz in Konzentrationen von 1mg/ml und 0,2mg/ml auf *Candida parapsilosis*:
*Candida parapsilosis* wurde gemäß in der Routine häufig verwendeter Methoden zunächst auf YGC-Agar herangezogen, und im Anschluss daran in Flüssigmedium (Sabouraud Bouillon) überführt und inkubiert.

Nach Bestimmung der Zellzahl mittels Neubauer Zählkammer wurden 9 Versuchsansätze (Hefekulturen) mit je 1 x 10⁶ Hefe-Zellen/ml hergestellt, wobei drei Kulturen kein Zeolithpulver zugesetzt wurde (Kontrolle), 3 Kulturen Zeolithpulver in der Menge von 1mg/ml (0,1% M/V) und weiteren 3 Kulturen Zeolithpulver in der Menge von 0,2mg/ml (0,02% M/V) zugesetzt wurde. Die Versuchskulturen wurden bei 30°C unter Schütteln inkubiert:
3 x 12ml Sabouraud Bouillon
3 x 12ml Sabouraud Bouillon und 0,1% Zeolith
3 x 12ml Sabouraud Bouillon und 0,02% Zeolith

Zur Ermittlung der Blindwerte wurden Aliquots aller Medien auch ohne Keim inkubiert und im Versuch mitgeführt.

Nach 60min., 120min. und danach alle weiteren 30min. wurden die Stoffwechselaktivitäten jeder Versuchskultur indirekt mittels gängigem Viabilitätstest (Resazurinassay) bestimmt. Die Messwerte der 3 Ansätze derselben Kondition wurden gemittelt und inklusive Standardabweichung in Fig. 6 (Kurven, von oben: 0%, 0,02% und 0,1% Zeolith) dargestellt.

Selbst in der geringsten eingesetzten Konzentration (0,02%), mittlere Linie in Fig. 6, wirkt die erfindungsgemäße Substanz deutlich wachstumsverzögernd auf *Candida parapsilosis.*

### Bakterielle Vaginose

Die bakterielle Vaginose stellt die häufigste Form der Vaginitis dar.

Sie tritt auf, wenn die gesunde H₂O₂ produzierende Vaginalflora (hauptsächlich diverse Vertreter der Lactobacilli) im Rahmen einer Dysbiose durch vorwiegend anaerobe Bakterien ersetzt wird. Zu diesen zählen vorwiegend *Gardnerella vaginalis* und *Atopobium vaginae*, aber auch Vetreter der Gattungen Megasphaera, Dialister, Mobiluncus, Prevotella und andere. Risikofaktoren, die eine bakterielle Vaginose begünstigen sind unter anderem:
Niedriger sozioökonomischer Status (--> Stress)
Häufige Vaginalspülungen
Rauchen
Ungeschützter Geschlechtsverkehr mit häufig wechselnden Partnern
Kontrazeptiva im Uterus
Häufige Verwendung höherer Dosen des Spermizids Nonoxynol-9

### Therapieempfehlungen:

Außerhalb der Schwangerschaft wird 7 Tage lang mit Metronidazol (2 x 500mg/Tag) therapiert. Weitere Möglichkeiten bestehen in der lokalen Verabreichung von Metronidazol oder Clindamycin in Form 2%iger Vaginalcremes (Zeitraum 7 Tage). Auch der Einsatz von Vaginaltabletten zur zweimaligen lokalen Verabreichung von 500mg Metronidazol pro Tag für den Zeitraum von 7 Tagen ist möglich. Darüber hinaus gibt es Ovula zur intravaginalen Anwendung von Clindamycin (100mg täglich für 3 Tage).

Der adhärente bakterielle Biofilm wird bei den beschriebenen Therapien jedoch nicht beseitigt, daher liegt die Heilungsquote nach 3 Monaten nur bei 60-70% und nach 6 Monaten noch weiter darunter. Durch den Einsatz von Probiotika, die u.a. den vaginalen pH-Wert absenken und die gesunde Vaginalflora fördern, kann die Rezidivquote der BV etwa um die Hälfte reduziert werden (siehe [2]: S1-Therapie der Vaginalmykose).

Die Wirksamkeit der erfindungsgemäßen Substanz geht aus den nachfolgend geschilderten Untersuchungen und Experimenten an *Gardnerella vaginalis* hervor:

### 5. Versuch:

Wirkung der erfindungsgemäßen Substanz in Konzentration 1mg/ml (0,1% M/V) und 10mg/ml (1% M/V) auf *Gardnerella vaginalis*:
*Gardnerella vaginalis* (DSM Nr. 4944) [5] wurde gemäß in der Routine häufig verwendeter Methoden auf Casman Agar herangezogen und danach in Flüssigmedium (TSB+5% Horse Serum) überführt.

Nach Bestimmung der Zellzahl mittels Thoma Zählkammer wurden 12 Versuchsansätze (Kulturen) mit je 1 x 10⁵ Zellen/ml hergestellt, wobei 4 Kulturen kein Zeolithpulver zugesetzt wurde (Kontrolle), 4 Kulturen Zeolithpulver in der Menge von 1mg/ml und weiteren 4 Kulturen Zeolithpulver in der Menge von 10mg/ml zugesetzt wurde. Die Versuchskulturen wurden bei 37°C unter anaeroben Bedingungen 15h lang unter Schütteln inkubiert.

Zur Ermittlung der Blindwerte wurden alle Medien auch ohne Keim im Versuch mitgeführt.

Danach wurden Aliquots der Kulturen zur Bestimmung der Stoffwechselaktivität mittels Viabilitätstest (Vialight-Assay) entnommen.

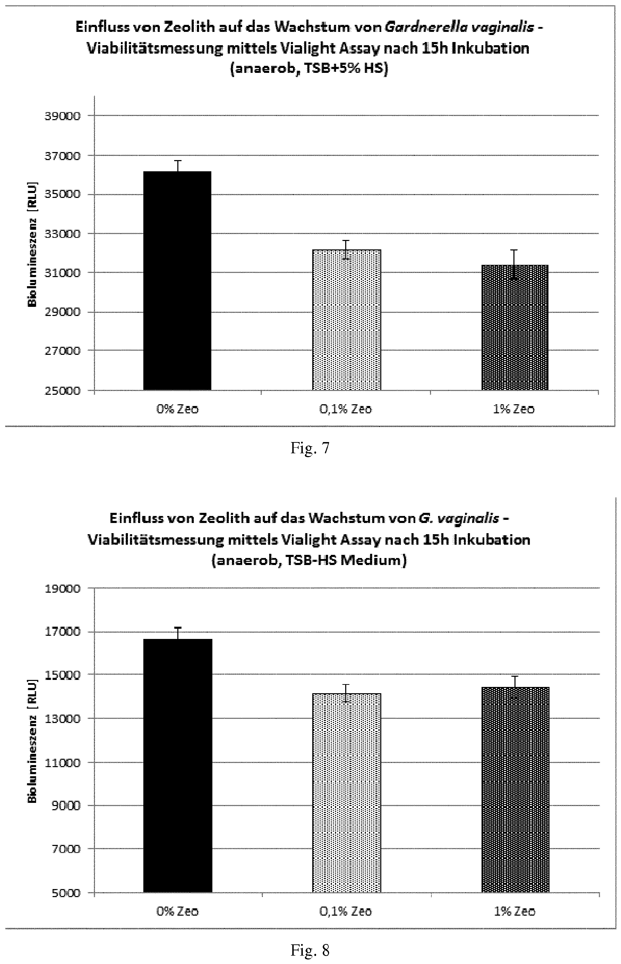

In Fig. 7 (Balken, von links: 0%, 0,1% und 1% Zeolith) sind die Ergebnisse grafisch dargestellt.

### 6. Versuch:

Der Wachstumstest wurde mit einer noch geringeren Start-Zellzahl (5 x 10⁴ Zellen/ml) wiederholt und lieferte ein vergleichbares Ergebnis, siehe Fig. 8 (Balken, von links: 0%, 0,1% und 1% Zeolith).

Die Förderung der erfindungsgemäßen Substanz auf das Wachstum der gesunden Vaginalflora geht aus nachfolgend dargelegten Untersuchungen und Experimenten, die an Bakterien der Gattung Lactobacillus durchgeführt wurden, hervor:

### 7. Versuch:

*Lactobacillus crispatus* (DSM Nr. 20584) [6] wurde gemäß in der Routine häufig verwendeter Methoden auf de Man Rogosa Sharp (MRS) Agar anaerob herangezogen und danach in Flüssigmedium (MRS Bouillon) überführt.

Nach Bestimmung der Zellzahl mittels Thoma Zählkammer wurden 12 Versuchsansätze (Kulturen) mit je 1 x 10⁶ Zellen/ml hergestellt, wobei 4 Kulturen kein Zeolithpulver zugesetzt wurde (Kontrolle), 4 Kulturen Zeolithpulver in der Menge von 1mg/ml (0,1% M/V) und weiteren 4 Kulturen Zeolithpulver in der Menge von 10mg/ml (1% M/V) zugesetzt wurde. Die Versuchskulturen wurden bei 37°C unter anaeroben Bedingungen unter Schütteln inkubiert.

Zur Ermittlung der Blindwerte wurden alle Medien auch ohne Keim im Versuch mitgeführt.

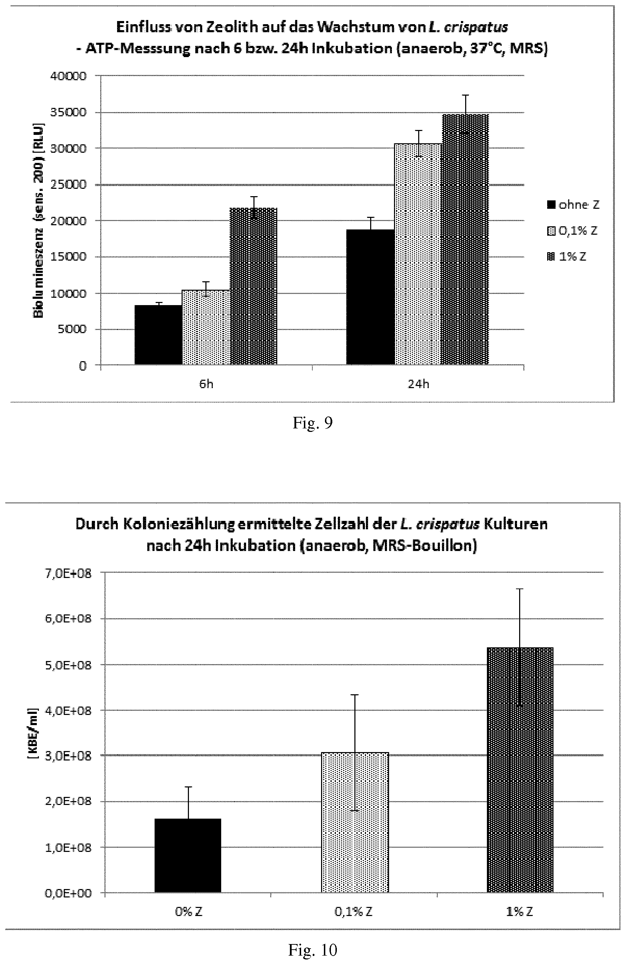

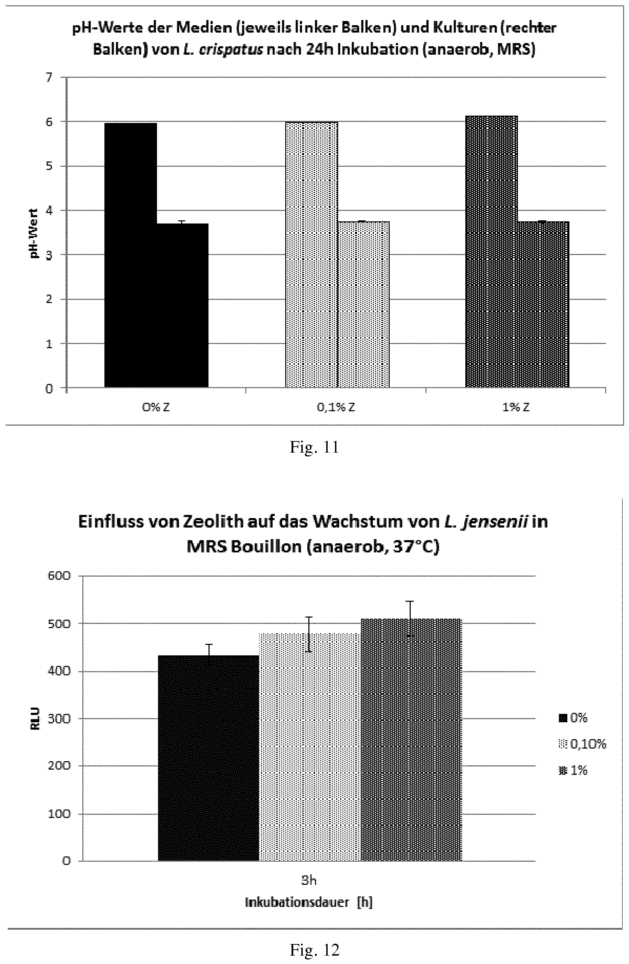

Nach 6 und 24 Stunden wurden Aliquots der Kulturen zur Bestimmung der Stoffwechselaktivität mittels Viabilitätstest (Vialight-Assay) entnommen, siehe Fig. 9 (Balken, von links, jeweils: 0%, 0,1% und 1% Zeolith). Nach 24h wurden darüber hinaus Aliquots der Kulturen verdünnt und auf MRS Agar ausplattiert, siehe Fig. 10 (Balken, von links: 0%, 0,1% und 1% Zeolith); und die pH-Werte der Versuchsansätze wurden zu Versuchsende mittels pH-Elektrode gemessen, siehe Fig. 11 (Balken, jeweils: Medien links, Kulturen rechts; jeweils von links: 0%, 0,1% und 1% Zeolith)

Die Ergebnisse der Koloniezählung (Fig. 10) bestätigten die Viabilitätsmessungen; die Fig. 11 zeigt die pH-Werte der Kulturen nach 24h Inkubation. Unabhängig davon, ob die erfindungsgemäße Substanz zugesetzt wurde oder nicht, in allen Fällen senkte der Keim den pH-Wert des Milieus auf unter 3,8 ab.

### 8. Versuch:

*Lactobacillus jensenii* (DSM Nr. 20557) [12] wurde gemäß in der Routine häufig verwendeter Methoden auf de Man Rogosa Sharp (MRS) Agar anaerob herangezogen und danach in Flüssigmedium (MRS Bouillon) überführt.

Nach Bestimmung der Zellzahl mittels Thoma Zählkammer wurden 12 Versuchsansätze (Kulturen) mit je 5 x 10⁵ Zellen/ml hergestellt, wobei 4 Kulturen kein Zeolithpulver zugesetzt wurde (Kontrolle), 4 Kulturen Zeolithpulver in der Menge von 1mg/ml (0,1% M/V) und weiteren 4 Kulturen Zeolithpulver in der Menge von 10mg/ml (1% M/V) zugesetzt wurde. Die Versuchskulturen wurden bei 37°C unter anaeroben Bedingungen unter Schütteln inkubiert.

Zur Ermittlung der Blindwerte wurden alle Medien auch ohne Keim im Versuch mitgeführt.

Nach 3 Stunden wurden Aliquots der Kulturen zur Bestimmung der Stoffwechselaktivität mittels Viabilitätstest (Vialight Assay) entnommen. Die Fig. 12 zeigt grafisch das Ergebnis des Experiments. Es zeigt sich eine Erhöhung der Stoffwechselleistung durch Inkubation mit der erfindungsgemäßen Substanz.

### 9. Versuch:

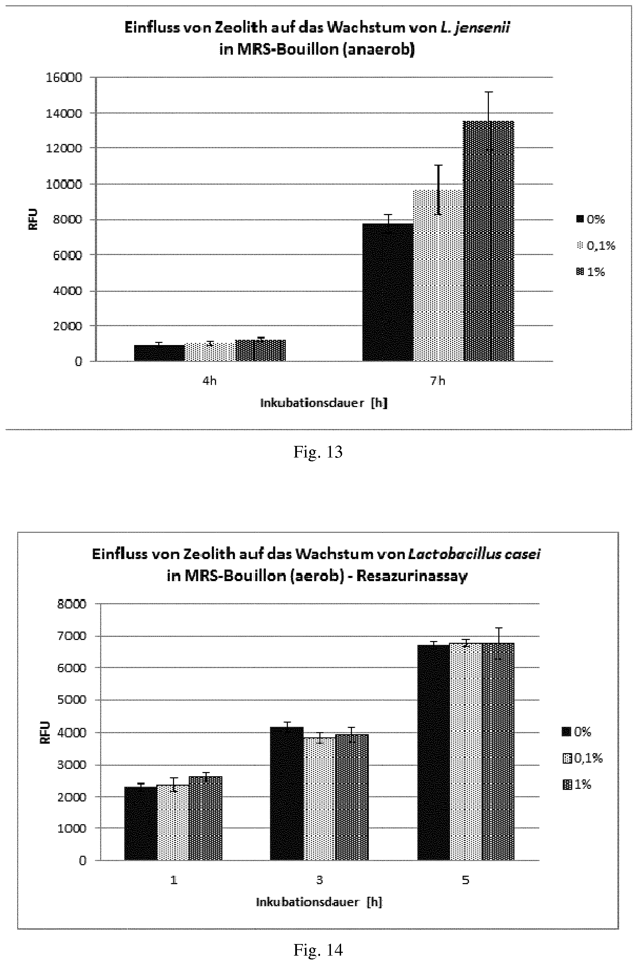

Das Wachstumsexperiment wurde unter Verlängerung der Inkubationsdauer wiederholt. Die Messung der Stoffwechselaktivität (Vialight Assay) erfolgte nach 4 und 7 Stunden auf gleiche Weise wie oben beschrieben. Die Fig. 13 zeigt die grafische Auswertung der Messergebnisse. Der wachstumsfördernde Effekt der erfindungsgemäßen Substanz auf *L*. *jensenii* wird nach 7 Stunden noch deutlicher sichtbar als nach 3 Stunden im Versuch 8.

### 10. Versuch:

*Lactobacillus casei* (DSM Nr. 20011) [11] wurde gemäß in der Routine häufig verwendeter Methoden auf de Man Rogosa Sharp (MRS) Agar aerob herangezogen und danach in Flüssigmedium (MRS Bouillon) überführt.
Nach Bestimmung der Zellzahl mittels Thoma Zählkammer wurden 12 Versuchsansätze (Kulturen) mit je 5 x 10⁵ Zellen/ml hergestellt, wobei 4 Kulturen kein Zeolithpulver zugesetzt wurde (Kontrolle), 4 Kulturen Zeolithpulver in der Menge von 1mg/ml (0,1% M/V) und weiteren 4 Kulturen Zeolithpulver in der Menge von 10mg/ml (1% M/V) zugesetzt wurde. Die Versuchskulturen wurden bei 37°C unter aeroben Bedingungen unter Schütteln inkubiert.
Zur Ermittlung der Blindwerte wurden alle Medien auch ohne Keim im Versuch mitgeführt.

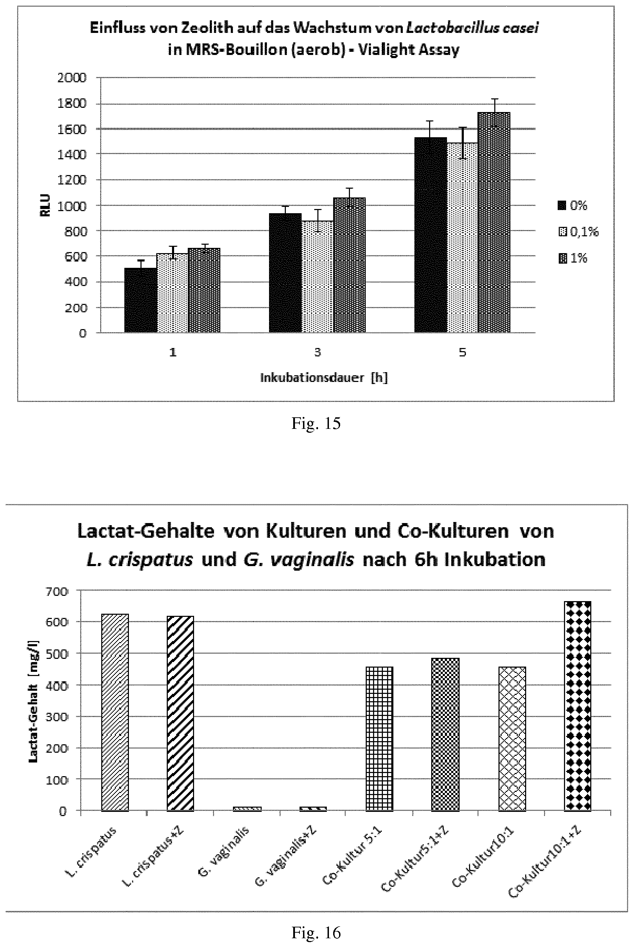

Nach 1, 3 und 5 Stunden wurden Aliquots der Kulturen zur Bestimmung der Stoffwechselaktivität mittels Viabilitätstest (Resazurin-Assay und Vialight-Assay) entnommen (siehe Fig. 14 und Fig. 15; beides Balken, von links: 0%, 0,1% und 1% Zeolith).

Die Ergebnisse in den Figura 14 und 15 zeigen überraschenderweise keinerlei Wirkung der erfindungsgemäßen Substanz auf die Stoffwechselaktivität des Versuchskeims innerhalb der Versuchsdauer. Weder mit Resazurin- noch mit ATP-Assay konnten über weite Strecken des Versuchs signifikante Unterschiede zwischen den einzelnen Konditionen gemessen werden.

Es ist überraschend, dass die erfindungsgemäße Substanz offensichtlich ganz spezifisch die für eine gesunde Vaginalflora relevanten Spezies *L. crispatus* und *L. jensenii* zwar im Wachstum fördert, gleichzeitig aber keinen Einfluss auf das Wachstum der für die Vaginalflora nicht relevanten Spezies *L. casei* ausübt.

Die folgenden Untersuchungen und Experimente belegen, dass die erfindungsgemäße Substanz in Kombination mit einem vaginalen Lactobacillus-Stamm die Regenerierung des gesunden Vaginalmilieus fördert bzw. wachstumshemmend auf *Gardnerella vaginalis,* einen der Hauptpathogene der Vaginose, wirkt.

### 11. Versuch:

*Lactobacillus crispatus* (DSM Nr. 20584) [6] und *Gardnerella vaginalis* (DSM Nr. 4944) [5] wurden gemäß in der Routine häufig verwendeter Methoden zunächst in Reinkultur angezüchtet.

Nach Bestimmung der Zellzahl der Vorkulturen mittels Thoma Zählkammer wurden 12 Versuchsansätze (Kulturen bzw. Co-Kulturen) mit je 5 x 10⁵ Zellen/ml in einem Mischmedium (TSB+HS und MRS im Verhältnis 7+3) hergestellt:
1 x 10ml Medium (7ml TSB + 5% Horse serum und 3ml MRS-Bouillon)
1x *G.vaginalis*
1x *G.vaginalis* + 0,5% Zeolith
1x *L.crispatus*
1x *L.crispatus* + 0,5% Zeolith
1x *L.crispatus* + *G.vaginalis* (5:1) A
1x *L.crispatus* + *G.vaginalis* + 0,5% Zeolith (5:1) A
1x *L.crispatus* + *G.vaginalis* (5:1) B
1x *L.crispatus* + *G.vaginalis* + 0,5% Zeolith (5:1) B
1x *L.crispatus* + *G.vaginalis* (10:1) A
1x *L.crispatus* + *G.vaginalis* + 0,5% Zeolith (10:1) A
1x *L.crispatus* + *G.vaginalis* (10:1) B
1x *L.crispatus* + *G.vaginalis* + 0,5% Zeolith (10:1) B

Die Versuchsansätze wurden bei 37°C unter anaeroben Bedingungen auf dem Orbitalschüttler inkubiert. Nach 6 und 24h wurden Aliquots der Kulturen abzentrifugiert (5min., 15000g), die Überstände 15min. lang bei 80°C im Wasserbad deaktiviert und einer enzymatischen Milchsäurequantifizierung [13] zugeführt. Die von den vaginalen Lactobacilli produzierte Milchsäure stellt einen der Hauptfaktoren des gesunden Vaginalmilieus dar.

Die Messergebnisse nach 6h Inkubation sind in Fig.16 grafisch dargestellt. Die Werte der Co-Kultur-Ansätze wurden für die Grafik jeweils gemittelt.

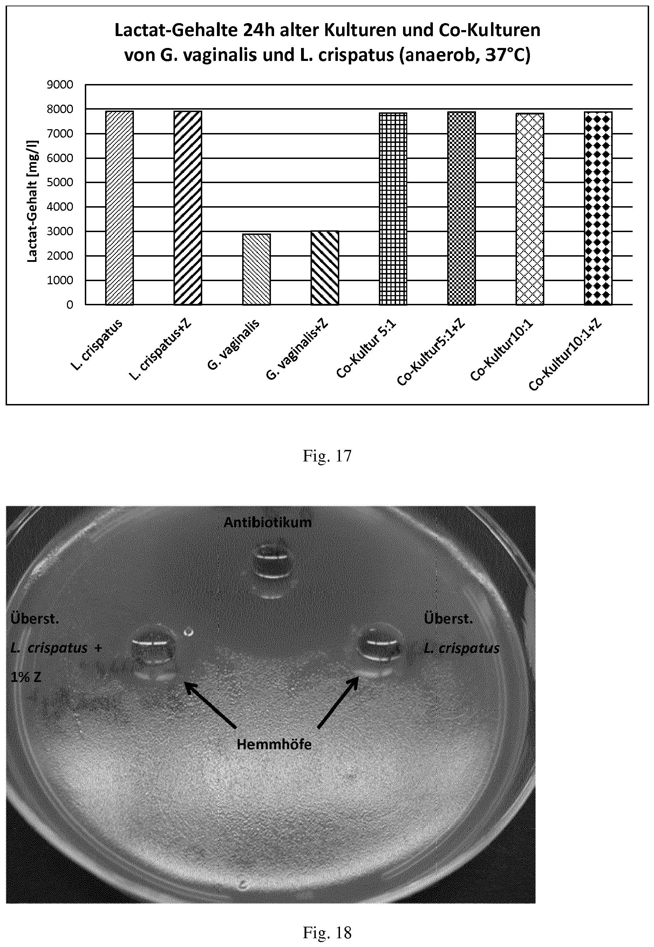

Anhand Fig. 16 wird deutlich, dass der Zusatz der erfindungsgemäßen Substanz zu den Co-Kulturen die Milchsäureproduktion von *L. crispatus* fördert.
In den gesättigten Kulturen nach 24h Inkubationszeit haben alle Co-kulturen annähernd gleich hohe Lactatgehalte wie die *L. crispatus* Reinkulturen (siehe Fig. 17), was darauf hindeutet, dass dieser Keim milieubestimmend wurde.

### 12. Versuch:

*Lactobacillus crispatus* (DSM Nr. 20584) [6] und *L. jensenii* (DSM Nr. 20557) [12] wurden gemäß in der Routine häufig verwendeter Methoden in Flüssigmedium anaerob kultiviert. Als Nährmedium wurde MRS-Bouillon ohne und mit Zusatz der erfindungsgemäßen Substanz (10mg/ml) herangezogen, welche vor dem Experiment im gleichen Medium vorkonditioniert wurde. Aliquots der gesättigten Kulturen wurden abzentrifugiert und die Überstände wie im Folgenden beschrieben auf ihre wachstumshemmende Wirkung auf *Gardnerella vaginalis* getestet.
*G.vaginalis* wurde über Nacht in TSB+5% horse serum angezüchtet und dicht auf BHI+5% horse serum-Agarplatten ausplattiert. Anschließend wurden mit einer sterilen Pipettenspitze je 3 Löcher in die Platten gestanzt. In diese Aussparungen der Agarplatten wurden die Überstände der *Lactobacillus*-Kulturen pipettiert. Als Positivkontrolle diente ein Mix aus Antibiotika (Penicillin/Streptomycin), als Negativkontrolle wurde Medium (zentrifugierte MRS-Bouillon) herangezogen. Die Platten wurden anaerob bei 37°C bebrütet, bis Rasenwachstum von *G*. *vaginalis* erkennbar war. Um die Luftfeuchtigkeit zu erhöhen, wurden den Inkubationskontainern feuchte Tücher zugegeben.

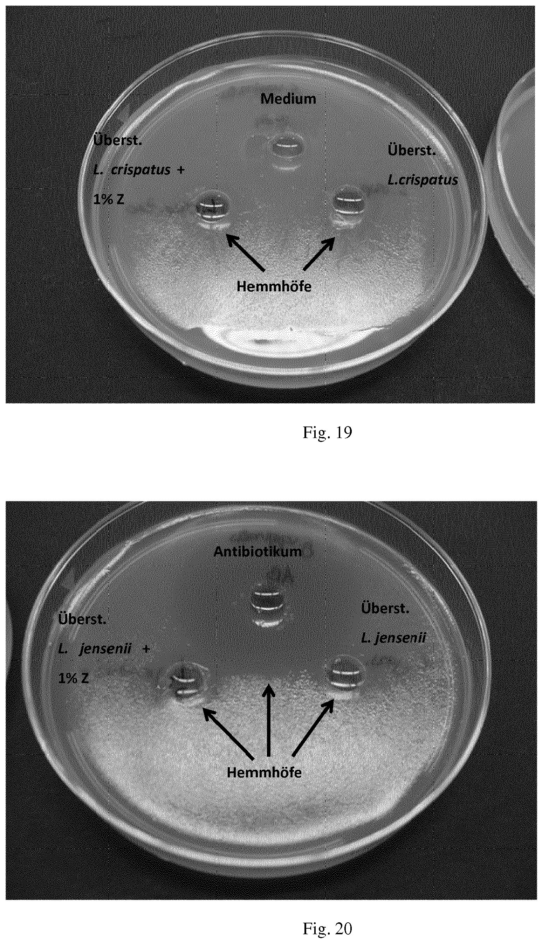

Bei der Auswertung zeigte sich eine Hemmung des Wachstums von *Gardnerella vaginalis* in Form von Hemmhöfen, die rund um die Aussparungen mit den Kulturüberständen sichtbar wurden. Dieser Effekt ist im Fall beider getesteter *Lactobacillus*-Stämme sowohl bei den Überständen mit als auch ohne Zusatz der erfindungsgemäßen Substanz feststellbar, allerdings bei *L. crispatus* stärker ausgeprägt als bei *L. jensenii.* Die folgenden Abbildungen (Figs. 18, 19 und 20) zeigen beispielhaft die Agarplatten zum Zeitpunkt der Versuchsauswertung. Der dichte Bakterienrasen von *Gardnerella vaginalis* ist rund um die Überstände der *Lactobacillus*-Kulturen (und der als Positivkontrolle mitgeführten Antibiotika) ausgespart.

Zusammenfassend kann festgestellt werden, dass einerseits eine Hemmung beziehungsweise die negative Beeinflussung des Wachstums potentiell pathogener Vaginalkeime durch die erfindungsgemäße Substanz gezeigt werden konnte. Andererseits konnte dokumentiert werden, dass relevante Keime des gesunden Vaginalmikrobioms im Wachstum nicht nur nicht beeinträchtigt, sondern sogar gefördert werden. Diese Wachstumshemmende Wirkung der erfindungsgemäßen Substanz auf pathogene Vaginalkeime bei gleichzeitiger tendenzieller und spezifischer Förderung des gesunden Vaginalmikrobioms kann als überraschend und neu angesehen werden.

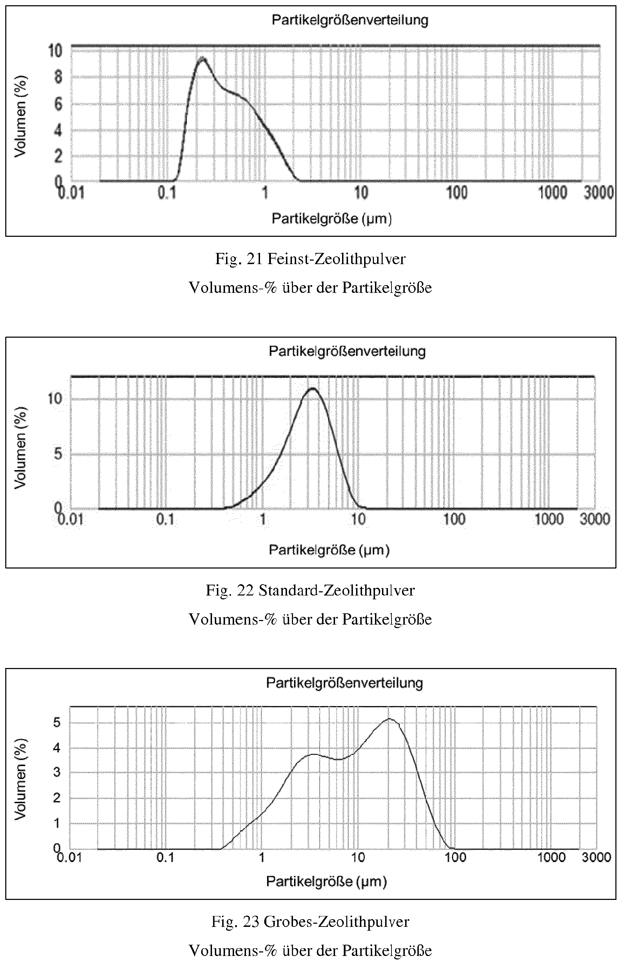

Zu der Wirkung der Korngrößenverteilung des erfindungsgemäßen Zeolithpulvers wurden drei verschiedene Batches für die im Folgenden beschriebenen Vergleichsversuche hergestellt. Dabei wird die Verteilung entsprechend Fig. 21 als "Feinst-Zeolithpulver", die Verteilung entsprechend Fig. 22 als "Standard-Zeolithpulver" und die Verteilung entsprechend Fig. 23 als "Grobes-Zeolithpulver" bezeichnet. Die tabellarischen Daten sind dabei die Folgenden:

**"Feinst-Zeolithpulver"**

| | **d (0.1) _{[µm]}** | **d (0.5) _{[µm]}** | **d (0.9) _{[µm]}** | **Residual** |
|---|---|---|---|---|
| **MW** | 0,19 | 0,38 | 1,06 | 5,36 |
| **s** | 0,00 | 0,00 | 0,01 | 0,14 |
| **RSD** | 0,54 | 1,13 | 1,37 | 2,69 |

**"Standard-Zeolithpulver" (wie auch in den Versuchen 1-12 verwendet**

| **008-02-08-4-0-0** | **d (0.1) _{[µm]}** | **d (0.5) _{[µm]}** | **d (0.9) _{[µm]}** | **Residual** |
|---|---|---|---|---|
| **MW** | 1,35 | 3,09 | 5,85 | 0,98 |
| **s** | 0,01 | 0,01 | 0,01 | 0,05 |
| **RSD** | 0,54 | 0,25 | 0,14 | 5,04 |

**"Grobes-Zeolithpulver"**

| | **d (0.1) _{[µm]}** | **d (0.5) _{[µm]}** | **d (0.9) _{[µm]}** | **Residual** |
|---|---|---|---|---|
| **MW** | 1,63 | 9,51 | 35,66 | 0,56 |
| **s** | 0,00 | 0,05 | 0,24 | 0,02 |
| **RSD** | 0,15 | 0,56 | 0,66 | 2,93 |

Es entspricht somit das "Feinst-Zeolithpulver" dem unteren erfindungsgemäßen Bereich, das "Standard-Zeolithpulver" dem oberen erfindungsgemäßen Bereich und das "Grobe-Zeolithpulver" liegt mit etwa 50% seiner Masse außerhalb (oberhalb) des erfindungsgemäßen Bereiches.

Die Versuchsdurchführung war die Folgende:
*Candida albicans* wurde gemäß in der Routine häufig verwendeter Methoden in Flüssigkultur (Sabouraud Bouillon) kultiviert. Nach Bestimmung der Zellzahl mittels Thoma Zählkammer wurden 12 Versuchsansätze (Kulturen) mit je 1 x 10⁵ Zellen/ml hergestellt, wobei 3 Kulturen kein Zeolithpulver zugesetzt wurde (Kontrolle), 3 Kulturen Zeolithpulver der Standardgrößenverteilung in der Menge von 10mg/ml, weiteren 3 Kulturen Feinst-Zeolithpulver in der Menge von 10mg/ml und abermals 3 Kulturen grobes Zeolithpulver in der Menge vom 10mg/ml. Die Versuchskulturen wurden bei 37°C unter aeroben Bedingungen unter Schütteln inkubiert.

Zur Ermittlung der Blindwerte wurden alle Medien auch ohne Keim im Versuch mitgeführt.

Über den Zeitraum von 7 Stunden wurden stündlich Aliquots der Kulturen zur Bestimmung der Stoffwechselaktivität mittels Viabilitätstest (Resazurin-Assay) entnommen. Bei dem Resazurin-Assay handelt es sich um einen in der biomedizinischen Forschung häufig eingesetzten Test zur Messung der Zytotoxizität von Substanzen. Es wird den Kulturproben der Redoxindikator Resazurin zugesetzt, welcher durch das im Stoffwechsel der Zellen generierte NADH zu fluoreszierendem Resorufin reduziert wird, dessen Konzentration fluorimetrisch bestimmt wird [7].

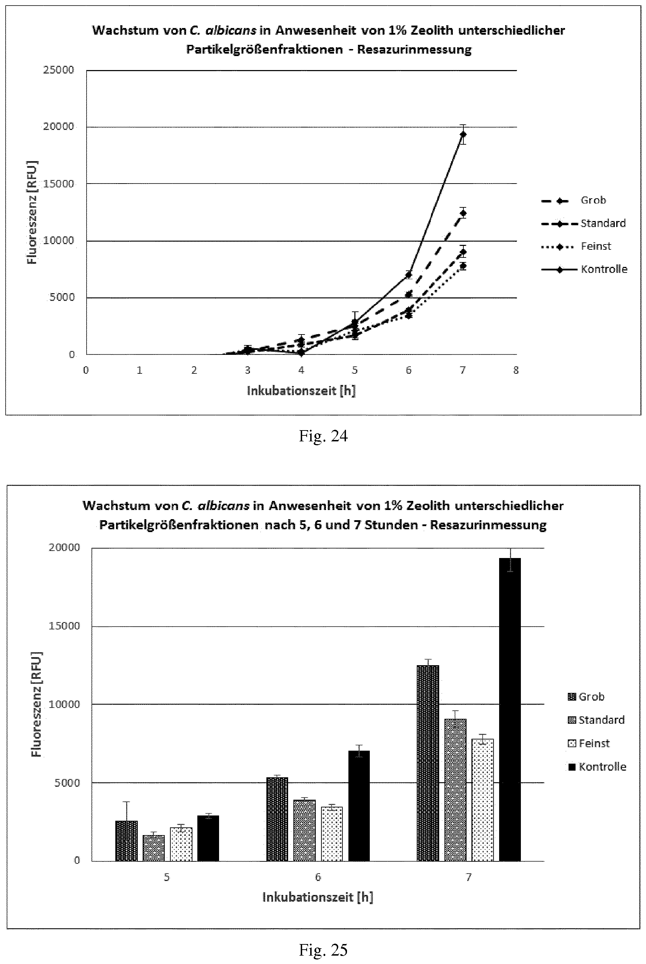

In Fig. 24 sind die Ergebnisse grafisch dargestellt. Es ist deutlich zu erkennen, dass die Wirkung von Zeolithpulver auf das Wachstum des Versuchskeims von der Partikelgröße stark abhängig ist. So zeigen die Versuchsansätze mit Feinst-Zeolithpulver (punktierte Kurve, im rechten Diagrammbereich die unterste Kurve) und Standard-Zeolithpulver (kurz strichlierte Kurve, die nächste oberhalb der Feinst-Zeolith-Kurve) eine signifikant stärkere Hemmung der Stoffwechselleistung als die grobe Partikelgrößenfraktion (lang strichlierte Kurve, die wiederum nächste oberhalb). Die oberste Kurve, entsprechend der Kontrollgruppe, erreicht dabei erheblich früher die Phase des (auch stärker ausgeprägten) exponentiellen Wachstums.

Die Fig. 25 zeigt die Messwerte nach 5, 6 und 7h Inkubation in Form von Balkendiagrammen. Dabei sind jeweils von links nach rechts das Grobe-Zeolithpulver, das Standard-Zeolithpulver, das Feinst-Zeolithpulver und die Kontrollgruppe angeordnet. Wie deutlich zu sehen ist, liegen die Werte der groben Partikelgrößenfraktion (ganz links) jenen der Kontrollen ohne Zeolith (ganz rechts) am nächsten.

Um zu prüfen, ob die unterschiedliche Wirkung der eingesetzten Zeolithpartikelgrößen auch für andere vaginale Pathogene aus der Gattung Candida gezeigt werden kann, wurden folgende Experimente an *Candida glabrata* [ATCC 2001], einem weiteren Keim der Vulvovaginalcandidose, durchgeführt:
Die Versuchsdurchführung war die Folgende:
*Candida glabrata* wurde gemäß in der Routine häufig verwendeter Methoden in Flüssigkultur (Sabouraud Bouillon) kultiviert. Nach Bestimmung der Zellzahl mittels Thoma Zählkammer wurden 12 Versuchsansätze (Kulturen) mit je 6 x 10⁵ Zellen/ml hergestellt, wobei 3 Kulturen kein Zeolithpulver zugesetzt wurde (Kontrolle), 3 Kulturen Zeolithpulver der Standardgrößenverteilung in der Menge von 10mg/ml, weiteren 3 Kulturen Feinst-Zeolithpulver in der Menge von 10mg/ml und abermals 3 Kulturen grobes Zeolithpulver in der Menge vom 10mg/ml. Die Versuchskulturen wurden bei 37°C unter aeroben Bedingungen unter Schütteln (110rpm) inkubiert.

Zur Ermittlung der Blindwerte wurden alle Medien auch ohne Keim im Versuch mitgeführt.

Über den Zeitraum von 7 Stunden wurden stündlich Aliquots der Kulturen zur Bestimmung der Stoffwechselaktivität mittels Viabilitätstest (Resazurin-Assay) wie oben beschrieben entnommen.

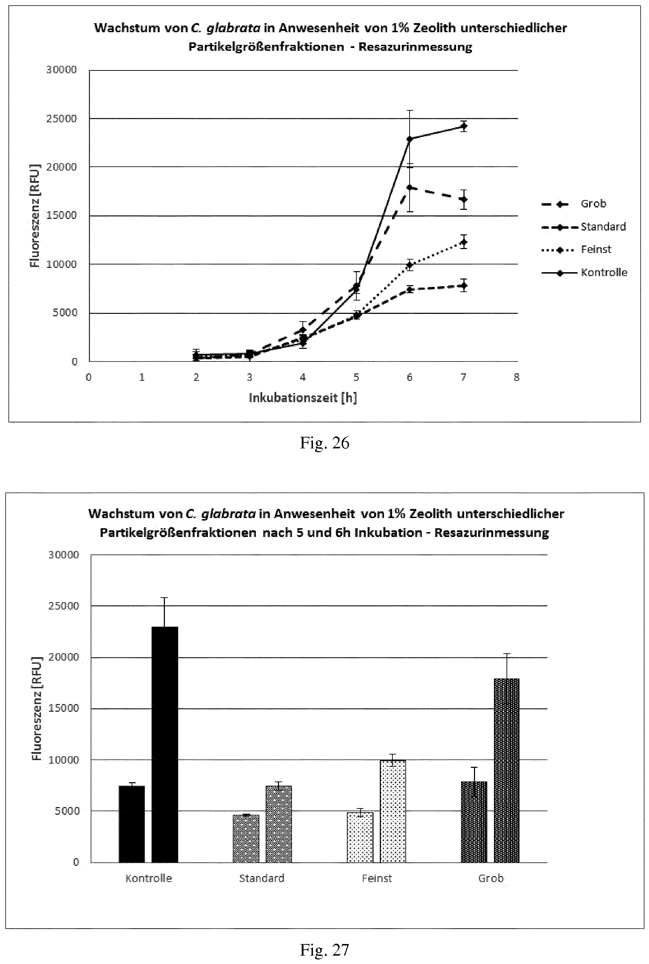

In Fig. 26 sind die Ergebnisse grafisch dargestellt. Auch für *C*. *glabrata* ist deutlich zu erkennen, dass die wachstumshemmende Wirkung von Zeolithpulver von der Partikelgröße stark beeinflusst wird. Wieder zeigen die Versuchsansätze mit Feinst-Zeolithpulver (punktierte Kurve, am rechten Rand die zweite von unten) und Standard-Zeolithpulver (kurz strichlierte Kurve, am rechten Rand ganz unten) eine deutlichstärkere Hemmung der Stoffwechselleistung als die grobe Partikelgrößenfraktion (lang strichlierte Kurve, zweite Kurve von oben).

Die Fig. 27 zeigt zur besseren Verdeutlichung der Ergebnisse die Messwerte nach 5 und 6h Inkubation in Form von Balkendiagrammen. Die Werte der groben Partikelgrößenfraktion (Balken ganz rechts im Diagramm) liegen jenen der Kontrollen ohne Zeolith (Balken ganz links im Diagramm) am nächsten. Die Standard-Zeolithgruppen sind links innen, die Feinst-Zeolithgruppen rechts innen abgebildet.

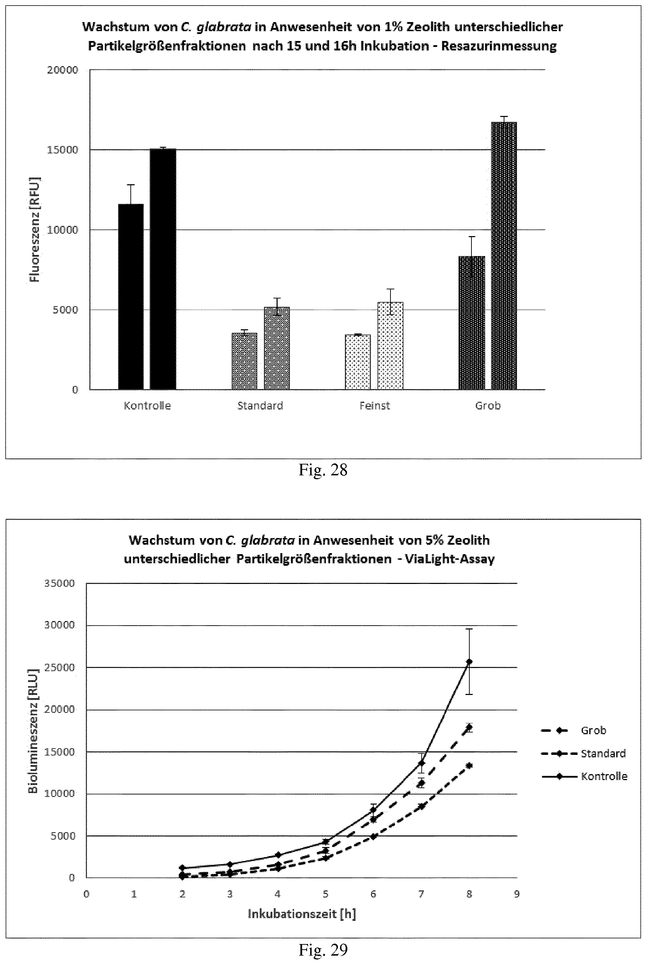

Um die zuvor dargestellten Untersuchungsergebnisse abzusichern, wurde ein wie zuvor beschriebener Wachstumsversuch durchgeführt, diesmal die Anzahl der eingesäten Zellen jedoch auf etwa 1 x 10⁴ Zellen/ml reduziert, dafür aber die Inkubationszeit auf 16 h ausgedehnt. Wie aus Fig. 28 ersichtlich, wurden Ergebnisse erzielt, die mit den Vorhergehenden sehr gut übereinstimmen.

Zur noch weiteren Absicherung der experimentell ermittelten Daten wurde ein wie zuvor beschriebener Wachstumsversuch durchgeführt, diesmal die Wirkkonzentration der eingesetzten Zeolithpulver jedoch auf 50mg/ml erhöht und mit dem Vialight Assay eine zweite Methode zur Bestimmung der Stoffwechselaktivität herangezogen. Dieser Test beruht auf der Quantifizierung des im Stoffwechsel von Zellen generierten Adenosintriphosphats (ATP) durch Messung der Biolumineszenz, welche bei Spaltung des ATP durch das Enzym Luciferase emittiert wird. [8]

Wie anhand der Wachstumskurven in Fig. 29 ersichtlich ist, wird auch unter diesen Versuchsbedingungen mit dem Standardprodukt (kurz strichlierte unterste, Kurve) ein deutlich stärkerer wachstums-hemmender Effekt erzielt als mit dem groben Zeolithpulver (lang strichlierte zweite Kurve von oben).

Wenn man zusammenfassend bedenkt, dass das verwendete "Grobe-Zeolithpulver" auch merkliche Anteile aus dem erfindungsgemäßen Größenbereich aufgewiesen hat, so ergibt sich eine sehr gute Grenze der Wirksamkeit am oberen erfindungsgemäßen Rand. Die Wirksamkeit des "Feinst-Zeolithpulvers" lässt die Grenze am unteren Rand erkennen, wo zufolge der Kleinheit der Partikel vermutlich die Struktur des Zeoliths beschädigt oder teilweise beschädigt ist.

Die Bestimmung der Korngröße ist nicht kritisch, bevorzugt erfolgt sie mit Hilfe eines Mastersizer 2000 der Malvern Instruments GmbH. Dieses Gerät arbeitet auf optischer Basis nach dem Prinzip der Beugung eines Laserstrahls. Dabei wird die Intensität des gestreuten Lichts eines Laserstrahls gemessen, der eine dispergierte und kontinuierlich bewegte Partikelprobe durchdringt. Vergleiche haben ergeben, dass so gemessene Korngrößen den real vorliegenden entsprechen.

Partikel, die in einer zu beurteilenden Zusammensetzung vorhanden sind, aber nicht im genannten und beanspruchten Korngrößenbereich liegen, werden als nicht vorhanden angesehen und nicht berücksichtigt.

Die Anwendung der erfindungsgemäßen Substanz kann in jeder pharmazeutisch unbedenklichen Zusammensetzung erfolgen, so können insbesondere eine oder mehrere der folgenden Zusatzstoffe verwendet werden: pharmazeutisch unbedenkliche Trägermaterialien; lebensfähige Mikroorganismen und/oder Extrakte daraus; Nährstoffe für das gesunde Vaginalmikrobiom (z.B. Lactose etc.); Stoffe, die das Vaginalmilieu für das gesunde Vaginalmikrobiom günstig beeinflussen, z.B. Estradiol, organische Säuren, etc..

Die Anwendung kann insbesondere in Form von Cremen, Salben, Gelen, Zäpfchen, Vaginaltabletten, Ovuli, Suppositorien, Schäumen, Aerosolen, Pulvern, Spülungen, Intimbädern, Suspensionen, etc. erfolgen.

In beiden Ausgestaltungen sind die anwendbaren Materialien und Verfahren aus dem Stand der Technik bekannt und können vom Pharmazeuten in Kenntnis der zu behandelnden Erkrankung (Anwendung) und der Erfindung problemlos ausgewählt und durchgeführt werden.

### Referenzen:

[1] Hainer, B.,L. (2011) Vaginitis: Diagnosis and Treatment; American Academy of Family Physicians; http://www.sonoma.edu/users/w/wilkosz/n540a-07/VaginitisDX and TX.pdf
[2] AWMF online: S1-Leitlinie: Bakterielle Vaginose (BV) in Gynäkologie und Geburtshilfe; AWMF - Leitlinien-Register Nr. 015/28; aktueller Stand: 07/2013.
[3] AWMF online: S2k-Leitlinie: Vulvovaginalkandidose; AWMF-Register Nr. 015/072; Stand 12/2013.
[4] *Candida albicans* DSM Nr. 1386 https://www.dsmz.de/catalogues/details/culture/DSM-1386.html?tx_dsmzresources_pi5%5BreturnPid%5D=304
[5] *Gardnerella vaginalis* DSM Nr. 4944 https://www.dsmz.de/catalogues/details/culture/DSM-4944.html?tx_dsmzresources_pi5%5BreturnPid%5D=304
[6*] Lactobacillus crispatus* DSM Nr. 20584 https://www.dsmz.de/catalogues/details/culture/DSM-20584.html?tx_dsmzresources_pi5%5BreturnPid%5D=304
[7] Rampersad, S., N. (2012) Multiple Applications of Alarm Blue as an Indicator of Metabolic Function and Cellular Health in Cell Viability Bioassays; Review, Sensors 2012, 12, 12347-12360.
[8] VialightTM MDA plus - High sensitivity microbial detection kit with extended signal stability; Lonza LT07-122.
[9] Protokoll zur mikrobiologischen Untersuchung S0811-01
[10] Protokoll zur mikrobiologischen Untersuchung S0903-19
[11] *Lactobacillus casei* DSM Nr. 20011 https://www.dsmz.de/catalogues/details/culture/DSM-20011.html?tx_dsmzresources_pi5%5BreturnPid%5D=304
*[12] Lactobacillus jensenii* DSM Nr. 20557 https://www.dsmz.de/catalogues/details/culture/DSM-20557.html?tx_dsmzresources_pi5%5BretumPid%5D=304
[13] EnzytecTM D/L Milchsäure Art.Nr. E1255 - Gebrauchsanleitung; r-Biopharm.
[14] *Candida glabrata* ATCC Nr. 2001 https://www.atcc.org/∼/ps/2001.ashx

## Patentansprüche

1. Klinoptilolith, der eine Partikelgröße zwischen 0,2 und 10 µm aufweist, zur Verwendung bei der Behandlung von Vaginalerkrankungen wie Bakterieller Vaginose, Vulvovaginaler Candidose und Trichomoniasis von Säugetieren und Menschen sowie zur Wiederherstellung des gesunden Vaginalmikrobioms.

2. Klinoptilolith zur Verwendung nach Anspruch 1, **dadurch gekennzeichnet, dass** er von Schwermetallen befreit ist.

3. Klinoptilolith zur Verwendung nach Anspruch 1 oder 2, **dadurch gekennzeichnet, dass** er mit einem oder mehreren der folgenden Zusatzstoffe verwendet wird:
- pharmazeutisch unbedenkliche Trägermaterialien,
- lebensfähige Mikroorganismen und/oder Extrakte daraus,
- Nährstoffe für das gesunde Vaginalmikrobiom (z.B. Lactose etc.),
- Stoffe, die das Vaginalmilieu für das gesunde Vaginalmikrobiom günstig beeinflussen (z.B. Estradiol, organische Säuren etc.).

4. Klinoptilolith zur Verwendung nach einem der Ansprüche 1 bis 3, **dadurch gekennzeichnet, dass** er in einer der folgenden Verabreichungsformen lokal angewendet wird: Schaum, Zäpfchen, Vaginaltablette, Ovulum, Gel, Aerosol, Pulver, Spülung, Intimbad, Creme/Salbe, Suspension.

## Claims

1. Clinoptilolite, having a particle size of between 0.2 and 10 µm for use in the treatment of vaginal disorders such as bacterial vaginosis, vulvovaginal candidiasis, and trichomoniasis of mammals and humans, and also for restoring the healthy vaginal microbiota.

2. Clinoptilolite according to Claim 1, **characterized in that** it has been freed from heavy metals.

3. Clinoptilolite according to Claim 1 or 2, **characterized in that** it is used with one or more of the following adjuvants:
- pharmaceutically acceptable carrier materials,
- viable microorganisms and/or extracts thereof,
- nutrients for the healthy vaginal microbiota (e.g. lactose, etc.),
- substances which favorably influence the vaginal environment for the healthy vaginal microbiota (e.g. estradiol, organic acids, etc.).

4. Clinoptilolite for the use according to any of Claims 1 to 3, **characterized in that** it is applied locally in one of the following administration forms: foam, suppository, vaginal tablet, ovule, gel, aerosol, powder, rinse, douche, cream/ointment, suspension.

## Revendications

1. Clinoptilolithe en particules de taille comprise entre 0,2 et 10 microns, pour utilisation dans le traitement d'affections vaginales, comme une vaginose bactérienne, une candidose vulvo-vaginale et une trichomonase, chez les mammifères et les humains, ainsi que pour la restauration d'un microbiome vaginal sain.

2. Clinoptilolithe pour utilisation conforme à la revendication 1, **caractérisée en ce qu'**elle ne contient pas de métaux lourds.

3. Clinoptilolithe pour utilisation conforme à la revendication 1 ou 2, **caractérisée en ce qu'**elle est utilisée avec un ou plusieurs des adjuvants suivants :
- matériaux supports sans inconvénients du point de vue pharmaceutique,
- microorganismes viables et/ou extraits de tels microorganismes,
- substances nutritives pour un microbiome vaginal sain (par exemple, lactose, etc.),
- substances influençant le milieu vaginal de manière favorable pour un microbiome vaginal sain (par exemple oestradiol, acides organiques, etc.).

4. Clinoptilolithe pour utilisation conforme à l'une des revendications 1 à 3, **caractérisée en ce qu'**elle est appliquée localement sous l'une des formes galéniques suivantes : mousse, suppositoire, comprimé vaginal, ovule, gel, aérosol, poudre, lavement, bain intime, crème/pommade, suspension.
